Europäisches Patentamt

European Patent Office

Office européen des brevets

(11) Numéro de publication : **0 368 697 B1**

# (12) FASCICULE DE BREVET EUROPEEN

(45) Date de publication du fascicule du brevet :
**16.09.92 Bulletin 92/38**

(51) Int. Cl.⁵ : **C07C 321/26**

(21) Numéro de dépôt : **89402791.1**

(22) Date de dépôt : **10.10.89**

(54) **Méthyl-2 tertiobutyl-5 thiophénol, son procédé de préparation et son application.**

(30) Priorité : **08.11.88 FR 8814572**

(43) Date de publication de la demande :
**16.05.90 Bulletin 90/20**

(45) Mention de la délivrance du brevet :
**16.09.92 Bulletin 92/38**

(84) Etats contractants désignés :
**AT BE CH DE ES FR GB GR IT LI LU NL SE**

(56) Documents cités :
**EP-A- 0 152 097**
**FR-A- 1 274 294**
**US-A- 3 076 851**
**US-A- 4 006 186**

(56) Documents cités :
**CHEMICAL ABSTRACTS, vol. 104, no. 8, 24 février 1986, page 568, résumé no. 59416j, Columbus, Ohio, US; & JP-A-60 121 443**
**TETRAHEDRON LETTERS, vol. 29, juin 1988, pages 2671-2674, Oxford, GB; D.H.R. BARTON et al.: "Telluramine derivatives asselective oxidants"**

(73) Titulaire : **SOCIETE FRANCAISE HOECHST TOUR ROUSSEL HOECHST 1 Terrasse Bellini F-92800 Puteaux (FR)**

(72) Inventeur : **Christidis, Yani**
**53 Boulevard de la Villette**
**F-75019 Paris (FR)**

(74) Mandataire : **Rinuy, Santarelli**
**14, avenue de la Grande Armée**
**F-75017 Paris (FR)**

## Description

La présente invention concerne le méthyl-2-tertiobutyl-5 thiophénol, son procédé de préparation et son application.

Dans l'industrie du caoutchouc ainsi que dans celle des plastiques, on utilise couramment des dérivés soufrés tels que des thiols, des sulfures, etc... comme agents plastifiants, peptisants, stabilisants ou comme agents de transfert de chaînes dans des réactions de polymérisation radicalaire. Parmi ces dérivés, les alkylmercaptans, les xylylmercaptans ou les alkylthiophénols ainsi que leurs mercaptides, sont largement employés. Ces dérivés soufrés, quoique actifs et économiques, possèdent une odeur intrinsèque soufrée persistante et désagréable, même à faibles doses, qu'ils communiquent aux polymères les contenant, ce qui limite leurs applications industrielles et même interdit leur emploi dans l'emballage alimentaire et notamment dans la fabrication de bouteilles plastiques pour eaux de table.

De plus, depuis quelques années, on traite par des radiations électromagnétiques du matériel et/ou des produits alimentaires pré-emballés avec des films plastiques afin de les stériliser, ce qui nécessite l'emploi d'emballages plastiques stabilisés pour prévenir leur décomposition intempestive. Afin de répondre aux besoins du marché, on recherche donc depuis de nombreuses années des stabilisants efficaces, économiques et inodores.

Or la Demanderesse a découvert avec étonnement que le méthyl-2 tertiobutyl-5 thiophénol, contrairement aux autres thiophénols alkylés, présente simultanément d'excellentes propriétés de stabilisation des films plastiques ainsi que de bonnes propriétés d'agent de transfert de chaîne tout en n'ayant, et surtout tout en ne transmettant lors de son emploi, aucune odeur soufrée désagréable.

Le méthyl-2 tertiobutyl-5 thiophénol est à 20°C, un liquide incolore, transparent, fluide, réfringent, soluble dans les solvants organiques et insoluble dans l'eau, présentant un indice de réfraction de 1,5480 et possédant une odeur de citronnelle. Il est peu sensible aux oxydants, notamment aux peroxydes minéraux tels que les peroxodisulfates d'ammonium ou de métal alcalin couramment utilisés comme amorceurs de réaction de polymérisation radicalaire.

Il est aisément obtenu par réduction du chlorure de tertiobutyl-4 toluènesulfonyle-2 soit par le couple zinc-acide sulfurique, soit par le couple acide iodhydrique-phosphore rouge.

Le chlorure de tertiobutyl-4 toluènesulfonyle-2 est cité dans la littérature en mélange avec le chlorure de tertiobutyl-3 toluènesulfonyle-2 (23/77 en poids). Ce mélange présente un point d'ébullition de $155 \pm 2°C$ sous 2,7 kPa (M. Tashiro et al, Org. Prep. Proced. Int. 1976, $\underline{8}$ (6) 249-262). Dans le cadre de la présente invention, le chlorure de tertiobutyle-4 toluènesulfonyle-2 a été obtenu par chlorosulfonation directe du paratertiobutyltoluène avec l'acide chlorosulfonique dans le trichloroéthylène selon la méthode classique (Wagner-Zook, Synthetic Organic chemistry, method 550, pg 822, Wiley, New-York, 1953).

Dans ses applications, notamment comme stabilisant de polymères de chlorure de vinyle, le méthyl-2 tertiobutyl-5 thiophénol est généralement utilisé à des doses inférieures ou égales à 5 % en poids par rapport au poids de polymère. Habituellement, pour une application donnée, notamment comme agent peptisant, et pour un résultat identique, en raison de sa grande efficacité, ses doses d'emploi sont moindres que celles de produits similaires tels que le dodécylmercaptan, les xylylmercaptans, les alkylthiophénols (cf., par exemple, aux brevets français N° 1.274.294 et 1.339.458 et aux demandes de brevets europeen N° 84.882, 152.097 et 177.784) et il conduit chaque fois à des produits finis inodores. Ainsi, par exemple, lors de son utilisation comme agent de transfert de chaînes dans la réaction de polymérisation radicalaire de l'acrylate de méthyle et bien qu'il soit utilisé à la dose de 4 % en poids par rapport au poids des monomères, le polymère obtenu est inodore alors que pour obtenir le même résultat de viscosité, il faut utiliser une dose double, soit 8 % en poids par rapport au poids des monomères, de dodécylmercaptan et le polymère obtenu est inutilisable en raison de sa forte odeur soufrée.

Les exemples suivants illustrent l'invention sans toutefois la limiter.

### Exemple 1

Dans une solution agitée de :
– 296,5 g (2 moles) de tertiobutyl-4 toluène, dans
– 1460 g de trichloroéthylène,
on introduit en 4 heures à la température ambiante :
– 700 g (6 moles) d'acide chlorosulfonique.

La température du milieu réactionnel se maintient entre 20°C et 32°C et l'on absorbe le chlorure d'hydrogène dégagé à l'aide de flacons laveurs contenant de l'eau.

On abandonne ensuite le milieu réactionnel sous agitation à la température ambiante pendant 3 heures puis on le décante. La phase organique est ensuite versée sous agitation dans 2 kg d'eau glacée, puis après décantation elle est lavée avec une solution aqueuse saturée d'hydrogénocarbonate de sodium jusqu'à obtention d'eau de lavage à pH 6 et elle est ensuite séchée sur chlorure de calcium anhydre et enfin le trichloroéthylène est éliminé par distillation sous vide. On recueille 461 g d'huile que l'on distille à $170 \pm 2°C$ sous un vide de 300 Pa. On isole ainsi 419,5 g de chlorure de tertiobutyl-4 toluènesulfonyle-2 sous forme d'un liquide mobile.

```
          Microanalyse

                                  S %          Cl %

              calculés        13.00        14,37

              trouvés         13,00        14,4
```

### Exemple 2

On introduit rapidement à 90°C et sous agitation, 500 g de zinc en poudre fine dans 3700 g (2930 ml) d'acide sulfurique 11,6N puis dans la suspension obtenue, on introduit à l'ébullition, en 45 minutes, 493 g (2 moles) de chlorure de tertiobutyl-4 toluènesulfonyle-2.

On abandonne ensuite le milieu réactionnel une heure au reflux puis on le refroidit à la température ambiante. On extrait le produit cherché au toluène puis les phases organiques toluéniques sont lavées successivement à l'eau puis avec une solution aqueuse saturée d'hydrogénocarbonate de sodium, et enfin, elles sont distillées sous vide.

On recueille ainsi 292 g (1,62 mole) de méthyl-2 tertiobutyl-5 thiophénol distillant à 121-122°C sous un vide de 1,9 kPa, soit un rendement de 81 % de la théorie calculée par rapport au chlorure de tertiobutyl-4 toluènesulfonyle-2 mis en oeuvre.

```
          Analyse physique RMN du proton (CDCl )
                                                  3
              1,30 ppm       s    9H     tertiobutyle

              2,30 ppl       s    3H     méthyle

              3,20 ppm       s    1H     thiophénol

              7-7,3 ppm      m    3H     aromatiques.

          Analyse physique :

          - chromatographie en phase gazeuse,

          - produit homogène.


          Microanalyse

                             C %        H %          S %

          C  H  S
           11 16
          180,31   calculés   73,27      8,94        17,78

                   trouvés    73,4       9,2         17,5
```

### Exemple 3

On émulsionne à la température ambiante :
– 320 g d'acrylate de méthyle,
– 12,8 g de méthyl-2 tertiobutyl-5 thiophénol, dans :
– 175 g d'eau déminéralisée,
– 8 g d'Hostapal BV conc., commercialisé par la Demanderesse,
– 3,2 g de nonylphénol polyéthoxylé à 30 moles d'oxyde d'éthylène,
– 1,6 g d'acétate de sodium,
– 6,4 g de peroxodisulfate d'ammonium.

Cette émulsion est ensuite introduite en 2 heures sous agitation dans une solution, maintenue à 86-88°C et constituée par :
– 200 g d'eau déminéralisée,
– 2,6 g d'Hostapal BV conc.,
puis, l'addition terminée, on poursuit le chauffage 30 minutes à 88°C et enfin on introduit en 90 minutes à cette température :
– 317 g de soude à 47 % en poids,
en distillant le méthanol formé. Lorsqu'on a recueilli 50 g de distillat, on refroidit le milieu réactionnel à la température ambiante. On obtient ainsi 1000 g d'une solution aqueuse de polyacrylate de sodium à 40 % en poids, présentant une viscosité Brookfield RVT, à la vitesse 100, de 200 mPa.s et ne possédant aucune odeur.

**Revendications**

1. Méthyl-2 tertiobutyl-5 thiophénol.

2. Procédé de préparation du méthyl-2 tertiobutyl-5 thiophénol, caractérisé en ce que l'on réduit le chlorure de tertiobutyl-4 toluènesulfonyle-2 avec le couple zinc-acide sulfurique.

3. Application du méthyl-2 tertiobutyl-5 thiophénol comme stabilisant des polymères de chlorure de vinyle.

4. Application du méthyl-2 tertiobutyl-5 thiophénol comme agent de transfert de chaîne.

5. Application du méthyl-2 tertiobutyl-5 thiophénol comme agent peptisant.

**Patentansprüche**

1. 2-Methyl-5-tert.butylthiophenol.

2. Verfahren zur Herstellung von 2-Methyl-5-tert.butylthiophenol, dadurch gekennzeichnet, daß man das 4-tert.Butyl-2-toluolsulfonylchlorid mit dem Paar Zink-Schwefelsäure reduziert.

3. Verwendung des 2-Methyl-5-tert.butylthiophenols als Stabilisator von Vinylchloridpolymeren.

4. Verwendung des 2-Methyl-5-tert.butylthiophenols als Kettenübertragungsmittel.

5. Verwendung des 2-Methyl-5-tert.butylthiophenols als Peptisiermittel.

**Claims**

1. Methyl-2 tertiobutyl-5 thiophenol.

2. A preparation process for Methyl-2-tertiobutyl-5-thiophenol, characterized in that tertiobutyl-4-toluenesulfonyl-2 is reduced with the zinc-sulphuric acid couple.

3. The use of methyl-2-tertiobutyl-5-thiophenol as a stabilizer for vinyl chloride polymers.

4. The use of methyl-2-tertiobutyl-5-thiophenol as a chain-transfer agent.

5. The use of methyl-2-tertiobutyl-5-thiophenol as a peptizer.